(19) Europäisches Patentamt
European Patent Office
Office européen des brevets

(11) EP 2 100 609 A1

(12) **EUROPEAN PATENT APPLICATION**
published in accordance with Art. 153(4) EPC

(43) Date of publication:
**16.09.2009 Bulletin 2009/38**

(21) Application number: **07850166.5**

(22) Date of filing: **06.12.2007**

(51) Int Cl.:
*A61K 31/4365* (2006.01) *A61K 9/14* (2006.01)
*A61K 9/16* (2006.01) *A61K 9/20* (2006.01)
*A61K 9/48* (2006.01) *A61K 47/10* (2006.01)
*A61K 47/26* (2006.01) *A61P 7/02* (2006.01)

(86) International application number:
**PCT/JP2007/073550**

(87) International publication number:
**WO 2008/072534 (19.06.2008 Gazette 2008/25)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **07.12.2006 JP 2006330374**

(71) Applicants:
- **Daiichi Sankyo Company, Limited**
**Chuo-ku**
**Tokyo 103-8426 (JP)**
- **Ube Industries, Ltd.**
**Ube-Shi,**
**Yamaguchi 755-8633 (JP)**

(72) Inventors:
- **WATANABE, Tomoyuki**
**Kanagawa 254-0014 (JP)**
- **MAEDA, Kazuko**
**254-0014 Kanagawa (JP)**

(74) Representative: **Fairbairn, Angus Chisholm**
**Marks & Clerk LLP**
**90 Long Acre**
**London**
**WC2E 9RA (GB)**

# (54) SOLID MEDICINAL PREPARATION CONTAINING MANNITOL OR LACTOSE

(57) A solid medicinal preparation containing the compound of the following general formula (I) or a pharmacologically acceptable salt thereof. It has improved content uniformity. The solid medicinal preparation contains: (A) the compound of the following general formula (I): or a pharmacologically acceptable salt thereof; and (B) mannitol or lactose which, when examined under specific conditions, has a particle size distribution in which the 90% cumulative diameter is 80 to 300 μm.

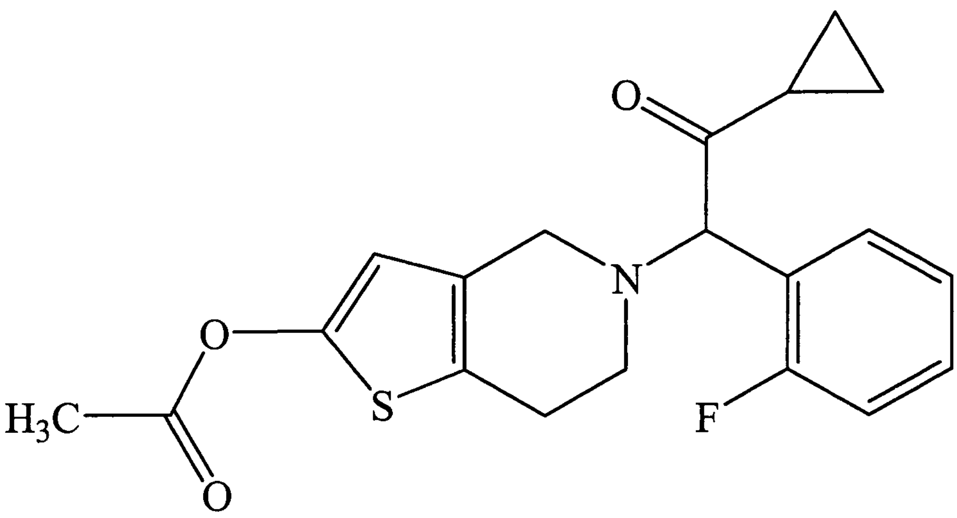

(I)

EP 2 100 609 A1

## Description

[TECHNICAL FIELD]

The present invention relates to a solid medicinal preparation containing

(A) a compound represented by the following general formula (I):

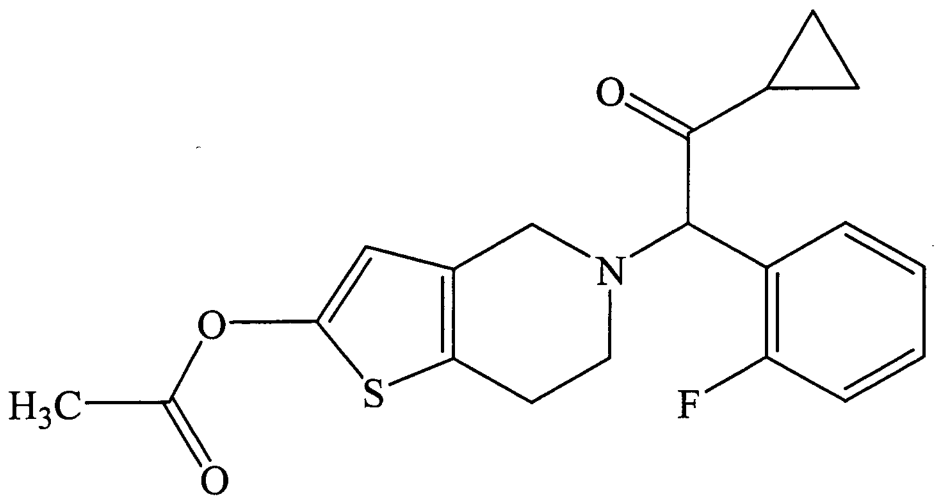

(I)

or a pharmacologically acceptable salt thereof; and
(B) mannitol or lactose which, when measured under the conditions described later, has a particle size distribution in which the 90% cumulative diameter is 80 to 300 μm.

[BACKGROUND ART]

The compound represented by the aforementioned general formula (I) or a pharmacologically acceptable salt thereof is known as a compound having platelet aggregation inhibition activity (Patent Document 1 or 2).
Patent Documents 2, 3, 4, 5, 6 and 7 exemplify various kinds of additives that may be used in preparations containing the compound represented by the aforementioned general formula (I) or a pharmacologically acceptable salt thereof, and there is a line which mentions lactose and/or mannitol as one such additive. Further, a preparation example which formulates lactose is disclosed.
However, none of these Patent Documents specifically discloses mannitol or lactose having a 90% cumulative diameter of 80 to 300 μm, and there is no disclosure or teaching that a composition containing the compound represented by the aforementioned general formula (I) or a pharmacologically acceptable salt thereof can have improved content uniformity by including mannitol or lactose having a 90% cumulative diameter of 80 to 300 μm.

[Patent Document 1] Japanese Patent Application (Kokai) No. Hei 6-41139
[Patent Document 2] Japanese Patent Application (Kokai) No. 2002-145883
[Patent Document 3] Japanese Patent Application (Kokai) No. Hei 10-310586
[Patent Document 4] Japanese Patent Application (Kokai) No. 2002-255814
[Patent Document 5] Japanese Patent Application (Kokai) No. 2003-246735
[Patent Document 6] Japanese Patent Application (Kokai) No. 2004-51639
[Patent Document 7] pamphlet of International Publication WO 2004/098713

[DISCLOSURE OF THE INVENTION]

[PROBLEMS TO BE SOLVED BY THE INVENTION]

An object of the present invention is to provide a solid medicinal preparation containing the compound of the aforementioned general formula (I) or a pharmacologically acceptable salt thereof, which has excellent content uniformity.

[MEANS FOR SOLVING THE PROBLEMS]

As a result of conducting extensive studies to solve the aforementioned problems, the inventors of the present invention found that a solid medicinal preparation containing the compound represented by the aforementioned general formula (I) or a pharmacologically acceptable salt thereof can have improved content uniformity by including mannitol or lactose which, when measured under specific conditions, has a particle distribution in which the 90% cumulative diameter is 80 to 300 μm, thereby leading to completion of the present invention.

The present invention provides a solid medicinal preparation containing (A) the compound represented by the aforementioned general formula (I) or a pharmacologically acceptable salt thereof; and (B) mannitol or lactose which, when measured under specific conditions, has a particle size distribution in which the 90% cumulative diameter is 300 μm or smaller (particularly a composition for prophylaxis or treatment of thrombosis or embolism), use of the compound represented by the aforementioned general formula (I) or a pharmacologically acceptable salt thereof for the production of the solid medicinal preparation (particularly a solid medicinal preparation for prophylaxis or treatment of thrombosis or embolism), and a prophylaxis or treatment strategy for a disease (particularly thrombosis or embolism) in which the solid medicinal preparation containing an effective amount of the compound represented by the aforementioned general formula (I) or a pharmacologically acceptable salt thereof is administered to a warm-blooded animal (particularly a human).

That is, the present invention is:

(1) a solid medicinal preparation comprising:

(A) a compound represented by the following general formula (I):

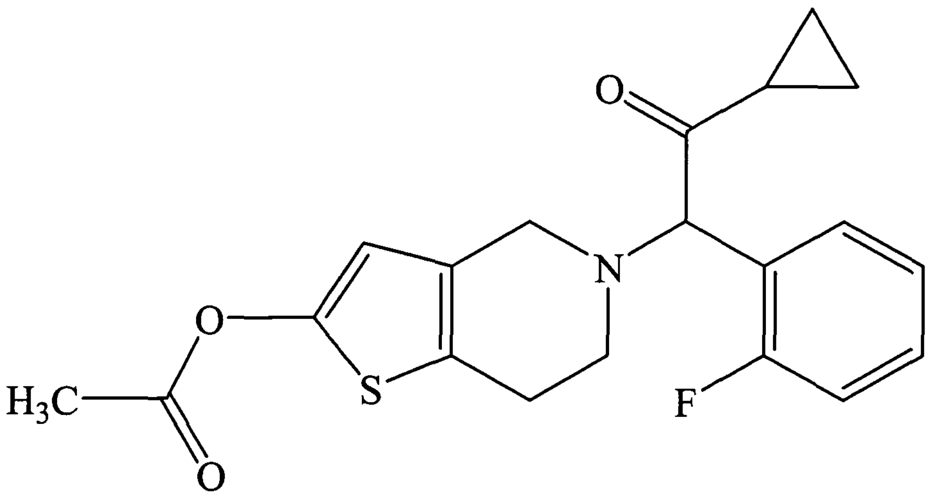

(I)

or a pharmacologically acceptable salt thereof; and

(B) mannitol or lactose which, when measured under the following conditions, has a particle size distribution in which the 90% cumulative diameter is 80 to 300 μm

(Measurement Conditions)
Particle size dispersion analyzer: HELOS (H1326) & RODOS system (manufactured by Sympatec GmbH);
Measurement range of laser diffraction unit: 0.5 to 875 μm; Calculation mode of laser diffraction unit: Fraunhofer HRLD (v3.2 Rel.2);
Dispersing apparatus: RODOS, Dry Dispersion System;
Dispersing pressure: 2.00 bar;
Vacuum degree: 100.00 mbar,
preferably,

(2) the solid medicinal preparation according to (1) comprising mannitol or lactose which has a particle size distribution in which the 90% cumulative diameter is 150 to 300 μm,

(3) the solid medicinal preparation according to (1) or (2) comprising mannitol,

(4) the solid medicinal preparation according to (1) or (2) comprising lactose,

(5) the solid medicinal preparation according to any one of (1) to (4), wherein the compound represented by the aforementioned general formula (I) or a pharmacologically acceptable salt thereof is a compound represented by the following formula (Ia):

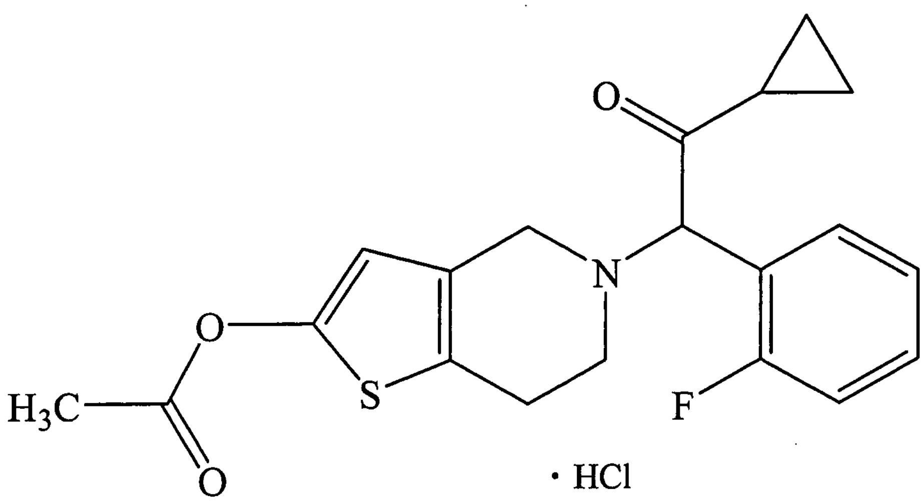

(Ia)

,

(6) the solid medicinal preparation according to any one of (1) to (5), wherein the preparation is in the form of powders, fine granules, granules, capsules or tablets,

(7) the solid medicinal preparation according to any one of (1) to (5), wherein the preparation is in the form of tablets, or

(8) the solid medicinal preparation according to any one of (1) to (7), characterized in that it is formed by a method comprising a step of formulating by direct compression.

[EFFECT OF THE INVENTION]

According to the present invention, a solid medicinal preparation containing the compound represented by the aforementioned formula (I) or a pharmacologically acceptable salt thereof which has excellent content uniformity can be provided.

The solid medicinal preparation of the present invention is, for example, useful for the treatment and/or prophylaxis of thrombosis or embolism (preferably thrombosis) and the like (preferably is a drug for the treatment and/or prophylaxis of thrombosis).

[BEST MODE FOR CARRYING OUT THE INVENTION]

The compound represented by the following general formula (I) :

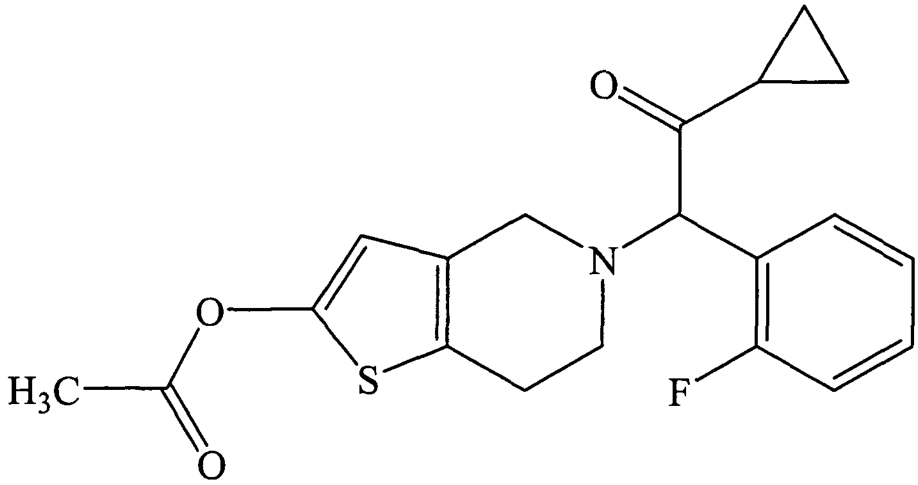

(I)

,

that is, 2-acetoxy-5-(α-cyclopropylcarbonyl-2-fluorobenzyl)-4,5,6,7-tetrahydrothieno[3,2-c]pyridine, or a pharmacologically acceptable salt thereof, which is the active ingredient of the solid medicinal preparation of the present invention, is disclosed in Japanese Patent Application (Kokai) No. Hei 6-41139 or Japanese Patent Application (Kokai) No. 2002-145883, and can be prepared accordingly.

As the "pharmacologically acceptable salt thereof" of the present invention, there may be mentioned for example, hydrohalides such as hydrofluoride, hydrochloride, hydrobromide or hydroiodide; inorganic acid salts such as nitrate, perchloric acid salt, sulfate or phosphate; lower-alkyl sulfonic acid salts such as methanesulfonate, trifluoromethanesulfonate or ethanesulfonate; aryl sulfonic acid salts such as benzenesulfonate or p-toluenesulfonate; organic acid salts such as acetate, malate, fumarate, succinate, citrate, ascorbate, tartrate, oxalate or maleate; or amino acid salts such as glycine salt, lysine salt, arginine salt, ornithine salt, glutamic acid salt or aspartic acid salt. The preferred salts are hydrohalides or organic acid salts, more preferably hydrochloride or maleate, and most preferably hydrochloride.

The "particle size distribution" of the "mannitol or lactose which has a particle size distribution in which the 90% cumulative diameter is 80 to 300 μm" of the present invention is measured under the following conditions.

(Measurement Conditions)

Particle size dispersion analyzer: HELOS (H1326) & RODOS System (manufactured by Sympatec GmbH);

Measuring range of laser diffraction unit: 0.5 to 875 μm; Calculation mode of laser diffraction unit: Fraunhofer HRLD (v3.2 Rel.2);

Dispersing apparatus: RODOS, Dry Dispersion System;

Dispersing pressure: 2.00 bar;

Vacuum degree: 100.00 mbar.

The "90% cumulative diameter" of the "mannitol or lactose which has a particle size distribution in which the 90% cumulative diameter is 80 to 300 μm" of the present invention means the particle diameter which corresponds to the cumulative amount of 90%, with respect to the curve obtained by taking particle diameter as the horizontal axis and taking the cumulative amount, obtained by cumulating the percentages of the particles from the small size, as the longitudinal axis. Such lactose is for example, Dilactose S (manufactured by Freund Corporation), Dilactose R (manufactured by Freund Corporation), Flowlac 100 (manufactured by MEGGLE AG) or Pharmatose DCL11 (manufactured by DMV-Fonterra fillers), and such mannitol is for example, Pearlitol 200SD (manufactured by Roquette) or the like. It is preferably mannitol or lactose having a 90% cumulative diameter of 150 to 300 μm, and most preferably lactose having a 90% cumulative diameter of 150 to 300 μm.

The solid medicinal preparation of the present invention may further contain additives such as appropriate pharmacologically acceptable lubricants, binders, emulsifiers, stabilizers, corrigents and/or diluents.

As the "lubricants" used, there may be mentioned for example, stearic acid; stearic acid metal salts such as calcium stearate or magnesium stearate; talc; colloidal silica; waxes such as beeswax or spermaceti; boric acid; adipic acid; sulfates such as sodium sulfate; glycol; fumaric acid; sodium stearyl fumarate; sucrose fatty acid esters; sodium benzoate; D,L-leucine; lauryl sulfates such as sodium lauryl sulfate or magnesium lauryl sulfate; silicates such as silicic anhydride or silicate hydrate; or starch derivatives such as corn starch, potato starch, α-starch or dextrin. Of these, stearic acid metal salts are preferably used.

As the "binders" used, there may be mentioned for example, hydroxypropyl cellulose, hydroxypropylmethyl cellulose, polyvinylpyrrolidone, polyethylene glycol, or compounds that can be used as fillers (for example, organic fillers including sugar derivatives such as lactose, sucrose, glucose, mannitol or sorbitol; starch derivatives such as corn starch, potato starch, $\alpha$-starch or dextrin; cellulose derivatives such as crystalline cellulose; gum Arabic; dextran; or pullulan: or inorganic fillers including silicate derivatives such as light anhydrous silicic acid, synthetic aluminum silicate, calcium silicate or magnesium metasilicate aluminate; phosphates such as calcium hydrogen phosphate; carbonates such as calcium carbonate; or sulfates such as calcium sulfate). Of these, hydroxypropyl cellulose or hydroxypropylmethyl cellulose is preferably used.

As the "emulsifiers" used, there may be mentioned for example, colloidal clays such as bentonite or beegum; metal hydroxides such as magnesium hydroxide or aluminum hydroxide; anionic surfactants such as sodium lauryl sulfate or calcium stearate; cationic surfactants such as benzalkonium chloride; or nonionic surfactants such as polyoxyethylene alkyl ether, polyoxyethylene sorbitan fatty acid ester or sucrose fatty acid ester.

As the "stabilizers" used, there may be mentioned for example, para-oxybenzoic acid esters such as methyl paraben or propyl paraben; alcohols such as chlorobutanol, benzyl alcohol or phenyl ethyl alcohol; benzalkonium chloride; phenols such as phenol or cresol; thimerosal; dehydroacetic acid; or sorbic acid.

As the "corrigents" used, there may be mentioned for example, sweeteners such as sodium saccharin or aspartame; acidulants such as citric acid, malic acid or tartaric acid; or flavorings such as menthol, lemon or orange.

Although there is no particular limitation as regards the amount of the compound represented by the aforementioned general formula (I) or a pharmacologically acceptable salt thereof formulated in the entirety of the solid medicinal preparation, it is preferable to formulate 1.0 to 30.0 % by weight (preferably 1.3 to 20.0 % by weight) with respect to the total weight of the solid medicinal preparation.

Although there is no particular limitation as regards the amount of additives formulated in the entirety of the solid medicinal preparation, it is preferable to formulate 10.0 to 93.5 % by weight (preferably 44.0 to 90.0 % by weight) of mannitol or lactose having a 90% cumulative diameter of 80 to 300 $\mu$m, 0.5 to 5.0 % by weight (preferably 0.5 to 3.0 % by weight) of lubricants, and 0.0 to 15.0 % by weight (preferably 2.5 to 10.0 % by weight) of binders, with respect to the total weight of the solid medicinal preparation.

As regards solid medicinal preparations of the present invention, there may be mentioned for example, tablets (including sublingual tablets and tablets that disintegrate in the mouth), capsules (including soft capsules and microcapsules), granules, fine granules, powders, pills, chewables or troches, preferably powders, fine granules, granules, capsules or tablets, and most preferably tablets.

As regards production methods for the solid medicinal preparation of the present invention, there may be used a general method described in publications such as "Powder Technology and Pharmaceutical Process (D. Chulia et al., Elservier Science Pub Co (December 1, 1993))". In particular, a dry method is preferable.

The dry method of the present invention includes a direct compression method and a dry granulation method, and a direct compression method is preferable.

The "direct compression method" is a method in which raw material powders are directly subjected to compression-molding to produce a preparation.

The "dry granulation method" is a method in which a preparation is produced using granules prepared by crushing and dividing by an appropriate method a compression-molded slug or sheet of raw material powders. These methods are described in publications such as "The Theory and Practice of Industrial Pharmacy (Third Edition) (Leon Lachman et al.: LEA & FEBIGER 1986)" and "Pharmaceutical Dosage Forms: Tablets volume 1 (Second Edition) (Herbert A. Lieberman et al.: MARCEL DEKKER INC. 1989)".

Granulation used here means an operation of forming granules having an almost uniform shape and size from a raw material in the form of powders, mass, solution, or molten liquid, and examples include granulation for forming a final product such as granules, powders or fine granules, and granulation for forming an intermediate product for the production of a tablet or a capsule.

The compression-molding process is a process in which a mass product of raw material powder is formed by applying pressure to the raw material powder using mechanical force, and examples include rotary tableting machines (manufactured by Kikusui Seisakusho Ltd., Hata Iron Works Co., Ltd., Sugawara Seiki Co., Ltd. and the like), and dry granulators such as a roller compactor, a roll granulator and a Chilsonator (manufactured by Freund Corporation, Turbo Kogyo Co., Ltd., Kurimoto, Ltd., Matsubo Corporation, Nippon Granulator Co., Ltd., Fuji Paudal Co., Ltd. and the like).

The crushing and dividing process is a process in which the mass product formed in the compression-molding process is crushed by means of a knife or cutter into an appropriate size, and examples of apparatuses used include mills and particle size selectors such as a power mill, Fitzmill, Fiore, and Co-mill (manufactured by Fuji Paudal Co., Ltd., Tokuju Corporation, Powrex Corporation and the like).

The thus obtained granulated product is subjected to particle size regulation so as to have a desired particle diameter, and then a preparation in the form of powders, fine granules or granules is produced. These preparations can also be produced as capsules by packing them in a capsule, or can be produced as tablets by further adding disintegrants

and/or lubricants if necessary and subjecting them to compression-molding by a tableting machine or the like. The operations of mixing and granulation are both widely used in the field of formulation techniques, and those skilled in the art can carry them out appropriately. In addition, tablets may be provided with at least one layer of a film-coating.

Coating is conducted by using a film-coating machine for example, and as the film coating base agent, there may be mentioned for example, sugar coating base agents, water-soluble film coating base agents, enteric film coating base agents or sustained release film coating base agents.

As the sugar coating base agents, saccharose is used, and one or more selected from talc, precipitated calcium carbonate, calcium phosphate, calcium sulfate, gelatin, gum Arabic, polyvinylpyrrolidone and pullulan can be used in combination.

As the water-soluble film coating base agents, there may be mentioned for example, cellulose derivatives such as hydroxypropyl cellulose, hydroxypropylmethyl cellulose, hydroxyethyl cellulose, methyl hydroxyethyl cellulose and sodium carboxymethyl cellulose; synthetic polymers such as polyvinyl acetal diethyl aminoacetate, aminoalkyl methacrylate copolymer and polyvinylpyrrolidone; and polysaccharides such as pullulan.

As the enteric film coating base agents, there may be mentioned for example, cellulose derivatives such as hydroxypropylmethyl cellulose phthalate, hydroxypropylmethyl cellulose acetate succinate, carboxymethylethyl cellulose or cellulose acetate phthalate; acrylic acid derivatives such as (meth)acrylic acid copolymer L, (meth)acrylic acid copolymer LD or (meth)acrylic acid copolymer S; or natural substances such as shellac.

As the sustained release film coating base agents, there may be mentioned for example, cellulose derivatives such as ethyl cellulose; or acrylic acid derivatives such as aminoalkyl methacrylate copolymer RS or ethyl acrylate-methyl methacrylate copolymer emulsion.

The aforementioned coating base agents may be used by combining two or more of them in an appropriate ratio. In addition, the coating base agents may, if necessary, further include additives such as appropriate pharmacologically acceptable plasticizers, fillers, lubricants, masking agents, colorants and/or antiseptics.

The plasticizers which may be used in the present invention are not particularly limited, and a person skilled in the art can select them appropriately. As for such plasticizers, there may be mentioned for example, propylene glycol, polyethylene glycol, polypropylene glycol, glycerin and sorbitol, glycerin triacetate, diethyl phthalate and triethyl citrate, lauric acid, sucrose, dextrose, sorbitol, triacetin, acetyl triethyl citrate, triethyl citrate, tributyl citrate or acetyl tributyl citrate.

As the masking agents which may be used in the present invention, there may be mentioned for example, titanium oxide.

As the colorants which may be used in the present invention, there may be mentioned for example, titanium oxide, iron oxide, red ferric oxide, yellow ferric oxide or yellow No. 5 aluminum lake talc.

As the antiseptics which may be used in the present invention, there may be mentioned for example, paraben.

The dosage amount of the compound represented by the aforementioned general formula (I) or a pharmacologically acceptable salt thereof, which is an active ingredient of the pharmaceutical composition of the present invention, may vary depending on various conditions such as the activity of the drug, symptoms, age or body weight of a patient. The daily dosage amount for an adult human has a lower limit of 0.01 mg (preferably 1 mg) and an upper limit of 200 mg (preferably 100 mg) in the case of oral administration.

[Examples]

The present invention will be described in more detail with reference to the Examples and Test Examples; however, the present invention shall not be limited to these.

Here, "Compound A" used in the Examples is the compound represented by the following formula (Ia):

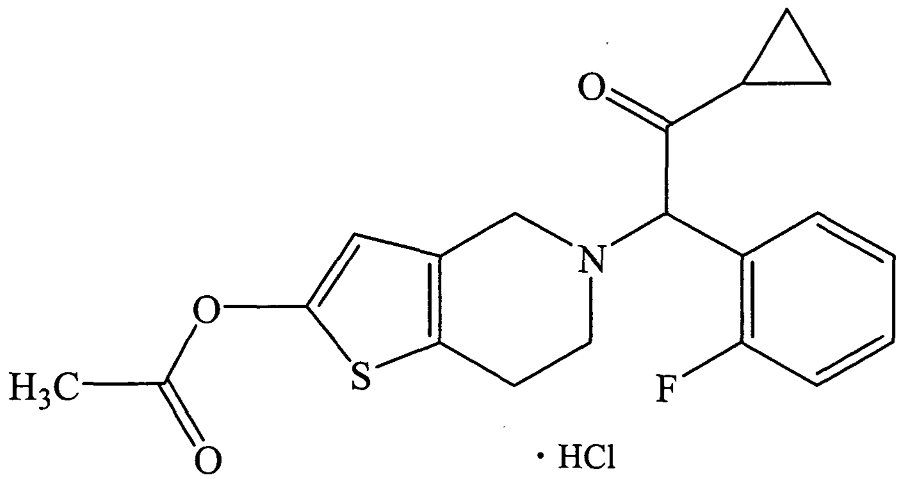

(Ia)

,

and can be prepared in accordance with the method disclosed in Japanese Patent Application (Kokai) No. 2002-145883.

(Example 1)

Compound A (14.3 g), hydroxypropyl cellulose (52.0 g), croscarmellose sodium (52.0 g) and lactose (Dilactose S, manufactured by Freund Corporation, 90% cumulative diameter: 164 μm) (916.5 g) were mixed using a high intensity mixer for 3 minutes, followed by addition of magnesium stearate (5.2 g), and the mixture was mixed again using the high intensity mixer to give a mixed powder.

The mixed powder obtained was compressed using a rotary type tableting machine with a tableting pressure of 5.9 kN so that the tablet mass became approximately 80 mg. The uncoated tablet obtained was subjected to film-coating in a pan-coating machine, by spraying a coating solution consisting of hydroxypropylmethyl cellulose, lactose, titanium oxide, triacetin and water, to give a tablet containing the test compound. Content uniformity testing was conducted on the obtained tablet. Test results are shown in Table 1.

(Example 2)

Compound A (14.3 g), hydroxypropyl cellulose (52.0 g), croscarmellose sodium (52.0 g) and lactose (Pharmatose DCL11, manufactured by DMV-Fonterra fillers, 90% cumulative diameter: 201 μm) (916.5 g) were mixed using a high intensity mixer for 3 minutes, followed by addition of magnesium stearate (5.2 g), and the mixture was mixed again using the high intensity mixer to give a mixed powder.

The mixed powder obtained was compressed using a rotary type tableting machine with a tableting pressure of 5.9 kN so that the tablet mass became approximately 80 mg. The uncoated tablet obtained was subjected to film-coating in a pan-coating machine, by spraying a coating solution consisting of hydroxypropylmethyl cellulose, lactose, titanium oxide, triacetin and water, to give a tablet containing the test compound. Content uniformity testing was conducted on the obtained tablet. Test results are shown in Table 1.

(Example 3)

Compound A (14.3 g), hydroxypropyl cellulose (52.0 g), croscarmellose sodium (52.0 g) and lactose (Flowlac 100, manufactured by MEGGLE AG, 90% cumulative diameter: 211 μm) (916.5 g) were mixed using a high intensity mixer for 3 minutes, followed by addition of magnesium stearate (5.2 g), and the mixture was mixed again using the high intensity mixer to give a mixed powder.

The mixed powder obtained was compressed using a rotary type tableting machine with a tableting pressure of 5.9 kN so that the tablet mass became approximately 80 mg. The uncoated tablet obtained was subjected to film-coating in a pan-coating machine, by spraying a coating solution consisting of hydroxypropylmethyl cellulose, lactose, titanium oxide, triacetin and water, to give a tablet containing the test compound. Content uniformity testing was conducted on the obtained tablet. Test results are shown in Table 1.

(Example 4)

Compound A (11.0 g), hydroxypropyl cellulose (40.0 g), croscarmellose sodium (40.0 g) and mannitol (Pearlitol 200SD, manufactured by Roquette, 90% cumulative diameter: 249 μm) (705.0 g) were mixed using a high intensity mixer for 3 minutes, followed by addition of magnesium stearate (4.0 g), and the mixture was mixed again using the high intensity mixer to give a mixed powder.

The mixed powder obtained was compressed using a rotary type tableting machine with a tableting pressure of 5.9 kN so that the tablet mass became approximately 80 mg. The uncoated tablet obtained was subjected to film-coating in a pan-coating machine, by spraying a coating solution consisting of hydroxypropylmethyl cellulose, lactose, titanium oxide, triacetin and water, to give a tablet containing the test compound. Content uniformity testing was conducted on the obtained tablet. Test results are shown in Table 1.

(Example 5)

Compound A (14.3 g), hydroxypropyl cellulose (52.0 g), croscarmellose sodium (52.0 g) and lactose (Dilactose R, manufactured by Freund Corporation, 90% cumulative diameter: 261 μm) (916.5 g) were mixed using a high intensity mixer for 3 minutes, followed by addition of magnesium stearate (5.2 g), and the mixture was mixed again using the high intensity mixer to give a mixed powder.

The mixed powder obtained was compressed using a rotary type tableting machine with a tableting pressure of 5.9 kN so that the tablet mass became approximately 80 mg. The uncoated tablet obtained was subjected to film-coating in a pan-coating machine, by spraying a coating solution consisting of hydroxypropylmethyl cellulose, lactose, titanium oxide, triacetin and water, to give a tablet containing the test compound. Content uniformity testing was conducted on the obtained tablet. Test results are shown in Table 1.

(Comparative Example 1)

Compound A (11.0 g), hydroxypropyl cellulose (40.0 g), croscarmellose sodium (40.0 g) and mannitol (Parteck M200, manufactured by Merck & Co., Inc., 90% cumulative diameter: 322 μm) (705.0 g) were mixed using a high intensity mixer for 3 minutes, followed by addition of magnesium stearate (4.0 g), and the mixture was mixed again using the high intensity mixer to give a mixed powder.

The mixed powder obtained was compressed using a rotary type tableting machine with a tableting pressure of 5.9 kN so that the tablet mass became approximately 80 mg. The uncoated tablet obtained was subjected to film-coating in a pan-coating machine, by spraying a coating solution consisting of hydroxypropylmethyl cellulose, lactose, titanium oxide, triacetin and water, to give a tablet containing the test compound. Content uniformity testing was conducted on the obtained tablet. Test results are shown in Table 1.

(Comparative Example 2)

Compound A (14.3 g), hydroxypropyl cellulose (52.0 g), croscarmellose sodium (52.0 g) and lactose (Tablettose 80, manufactured by MEGGLE AG, 90% cumulative diameter: 353 μm) (916.5 g) were mixed using a high intensity mixer for 3 minutes, followed by addition of magnesium stearate (5.2 g), and the mixture was mixed again using the high intensity mixer to give a mixed powder.

The mixed powder obtained was compressed using a rotary type tableting machine with a tableting pressure of 5.9 kN so that the tablet mass became approximately 80 mg. The uncoated tablet obtained was subjected to film-coating in a pan-coating machine, by spraying a coating solution consisting of hydroxypropylmethyl cellulose, lactose, titanium oxide, triacetin and water, to give a tablet containing the test compound. Content uniformity testing was conducted on the obtained tablet. Test results are shown in Table 1.

(Comparative Example 3)

Compound A (14.3 g), hydroxypropyl cellulose (104.0 g), croscarmellose sodium (52.0 g) and mannitol (Pearlitol 300DC, manufactured by Roquette, 90% cumulative diameter: 428 μm) (864.5 g) were mixed using a high intensity mixer for 3 minutes, followed by addition of magnesium stearate (5.2 g), and the mixture was mixed again using the high intensity mixer to give a mixed powder.

The mixed powder obtained was compressed using a rotary type tableting machine with a tableting pressure of 5.9 kN so that the tablet mass became approximately 80 mg. The uncoated tablet obtained was subjected to film-coating in a pan-coating machine, by spraying a coating solution consisting of hydroxypropylmethyl cellulose, lactose, titanium oxide, triacetin and water, to give a tablet containing the test compound. Content uniformity testing was conducted

on the obtained tablet. Test results are shown in Table 1.

(Test Example 1) Content Uniformity Test

One tablet was placed in a 20 ml amber volumetric flask. 14 ml of liquid for sample extraction was added to the flask, and then oscillation was applied until the tablet was completely disintegrated, by using an ultrasonic oscillation generating apparatus (medium: water at room temperature) and occasionally shaking the flask. The solution was centrifuged for 10 minutes at 3000 rpm, and the supernatant was used as the sample solution. The operation mentioned above was repeated 10 times and measurement was conducted on 10 tablets.
Separately, approximately 0.05 g of Compound A was precisely weighed and placed in a 50 ml amber volumetric flask. 40 ml of liquid for sample extraction was added to the flask, and then oscillation was applied to completely dissolve the tablet, by using an ultrasonic oscillation generating apparatus (medium: water at room temperature) and occasionally shaking the flask. Liquid for sample extraction was further added so that the solution became 50 ml. 5 ml of this solution was taken in a 100 ml amber volumetric flask, and liquid for sample extraction was added so that the solution became 100 ml. This solution was used as the standard solution.
Absorbance at 255 nm and 360 nm was measured using spectrophotometer UV-2500 or UV-3100 (manufactured by Shimadzu Corporation), with respect to the standard solution and sample solution. Cell length was 10 mm.

Liquid for sample extraction: solution mixture of acetonitrile / 0.002 mol/L potassium dihydrogenphosphate = 7/3 (v/v)

The content of each sample was calculated by the following equation.

Amount of compound represented by the general formula (I) ($C_{20}H_{20}FNO_3S$) with respect to the indicated amount (%)

$$= W \times \frac{373.44}{409.90} \times \frac{At_1 - At_2}{As_1 - As_2} \times \frac{1}{50} \times \frac{1}{0.001} \times 100$$

W: weighed amount of Compound A (g)
At1: absorbance of sample solution at 255 nm
At2: absorbance of sample solution at 360 nm
As1: absorbance of standard solution at 255 nm
As2: absorbance of standard solution at 360 nm
Relative standard deviation (RSD) was calculated from the content values obtained for each of the samples. Results are shown in Table 1.

(Test Example 2) Particle size distribution

Particle size distribution was measured for lactose and D-mannitol by laser diffraction method using HELOS (H1326) & RODOS System (manufactured by Sympatec GmbH). RODOS Dry Dispersion System was used to disperse the sample, and measurement was conducted under the following conditions. Measurement was conducted by n=3, and the average value of the observed particle diameter at 90% was obtained. Results are shown in Table 1.
HELOS (Laser diffraction unit):
Type: HELOS/KF
Lens: R5
Measurement range: 0.5 to 875 μm
Trigger conditions: 2s-100ms-k15-0.5%
Calculation mode: Fraunhofer HRLD (v3.2 Rel.2)
RODOS (Dispersing system):
Feeder: VIBRI
Dispersing pressure: 2.00 bar
Vacuum degree: 100.00 mbar

Rotation: 20.00 %
Feed: 60.00 %
Software: WINDOX Version 3.2 (manufactured by Sympatec GmbH)

(Table 1)

| Example | 90% cumulative diameter (μm) | RSD (%) |
|---|---|---|
| Example 1 | 164 | 0.7 |
| Example 2 | 201 | 1.4 |
| Example 3 | 211 | 1.5 |
| Example 4 | 249 | 1.0 |
| Example 5 | 261 | 1.3 |
| Comparative Example 1 | 322 | 3.8 |
| Comparative Example 2 | 353 | 3.0 |
| Comparative Example 3 | 428 | 2.7 |

From the aforementioned results, it is obvious that preparations of Examples 1 to 5 containing lactose or mannitol which, when measured under the conditions of Test Example 2, have a particle distribution in which the 90% cumulative diameter is 80 to 300 μm have excellent content uniformity, compared with the preparations of Comparative Examples 1 to 3 containing lactose or mannitol having a 90% cumulative diameter of 300 μm or larger.

[INDUSTRIAL APPLICABILITY]

According to the present invention, a pharmaceutical composition having improved content uniformity, which contains the compound represented by the aforementioned general formula (I) or the pharmacologically acceptable salt thereof, and lactose or mannitol, can be obtained.

## Claims

**1.** A solid medicinal preparation comprising:

(A) a compound represented by the following general formula (I):

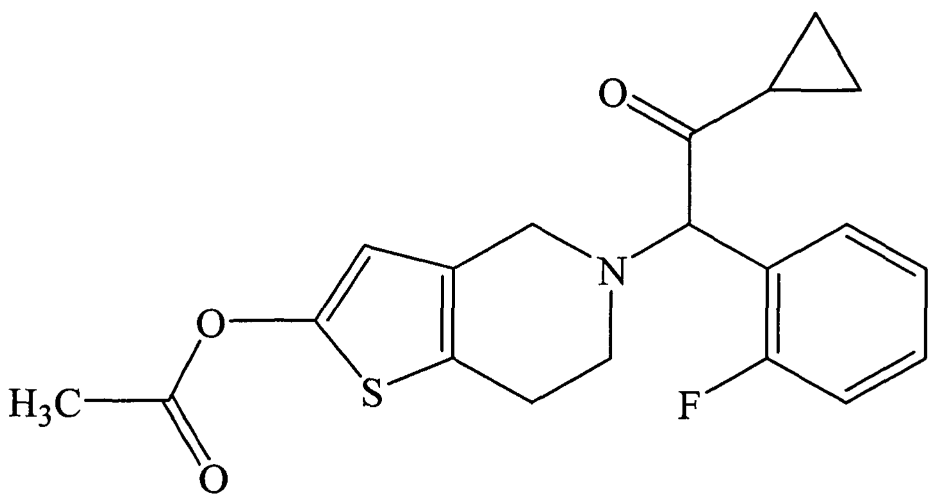

(I)

or a pharmacologically acceptable salt thereof; and
(B) mannitol or lactose which, when measured under the following conditions, has a particle size distribution in

which the 90% cumulative diameter is 80 to 300 μm

(Measurement Conditions)
Particle size dispersion analyzer: HELOS (H1326) & RODOS System (manufactured by Sympatec GmbH);
Measurement range of laser diffraction unit: 0.5 to 875 μm; Calculation mode of laser diffraction unit: Fraunhofer HRLD (v3.2 Rel.2);
Dispersing apparatus: RODOS Dry Dispersion System;
Dispersing pressure: 2.00 bar;
Vacuum degree: 100.00 mbar.

2. The solid medicinal preparation according to claim 1 comprising mannitol or lactose which has a particle size distribution in which the 90% cumulative diameter is 150 to 300 μm.

3. The solid medicinal preparation according to claim 1 or 2 comprising mannitol.

4. The solid medicinal preparation according to claim 1 or 2 comprising lactose.

5. The solid medicinal preparation according to any one of claims 1 to 4, wherein the compound represented by the general formula (I) or a pharmacologically acceptable salt thereof is a compound represented by the following formula (Ia):

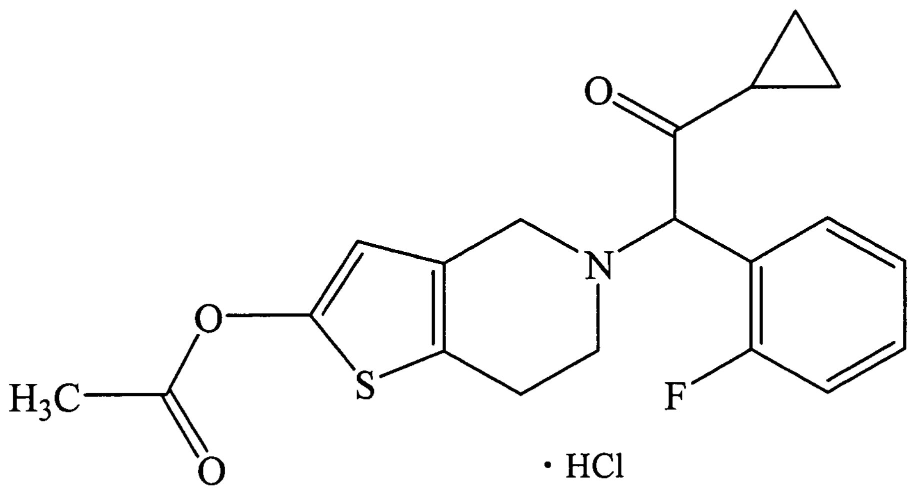

(Ia)

.

6. The solid medicinal preparation according to any one of claims 1 to 5, wherein the preparation is in the form of powders, fine granules, granules, capsules or tablets.

7. The solid medicinal preparation according to any one of claims 1 to 5, wherein the preparation is in the form of tablets.

8. The solid medicinal preparation according to any one of claims 1 to 7, **characterized in that** it is formed by a method comprising a step of formulating by direct compression.

**INTERNATIONAL SEARCH REPORT**

International application No.
PCT/JP2007/073550

A. CLASSIFICATION OF SUBJECT MATTER
*A61K31/4365*(2006.01)i, *A61K9/14*(2006.01)i, *A61K9/16*(2006.01)i, *A61K9/20*(2006.01)i, *A61K9/48*(2006.01)i, *A61K47/10*(2006.01)i, *A61K47/26*(2006.01)i, *A61P7/02*(2006.01)i
According to International Patent Classification (IPC) or to both national classification and IPC

B. FIELDS SEARCHED
Minimum documentation searched (classification system followed by classification symbols)
A61K31/4365, A61K9/14, A61K9/16, A61K9/20, A61K9/48, A61K47/10, A61K47/26, A61P7/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
REGISTRY/CAplus/EMBASE/BIOSIS/MEDLINE(STN), WPI

C. DOCUMENTS CONSIDERED TO BE RELEVANT

| Category* | Citation of document, with indication, where appropriate, of the relevant passages | Relevant to claim No. |
|---|---|---|
| Y | JP 2002-145883 A (Sankyo Co., Ltd.), 22 May, 2002 (22.05.02), Par. No. [0022]; pharmaceutical preparation examples 1 to 4 & WO 2002/04461 A1 & EP 1298132 A1 & US 2003/0134872 A1 & AU 2001067916 A | 1-8 |
| Y | JP 2003-246735 A (Sankyo Co., Ltd.), 02 September, 2003 (02.09.03), Par. No. [0023]; pharmaceutical preparation examples 1 to 4 (Family: none) | 1-8 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 April, 2008 (25.04.08) | 13 May, 2008 (13.05.08) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.
PCT/JP2007/073550

C (Continuation). DOCUMENTS CONSIDERED TO BE RELEVANT

| Category* | Citation of document, with indication, where appropriate, of the relevant passages | Relevant to claim No. |
|---|---|---|
| Y | JP 10-310586 A (Sankyo Co., Ltd.),<br>24 November, 1998 (24.11.98),<br>Par. No. [0029]; pharmaceutical preparation examples 1, 2<br>& WO 97/49397 A1 & AU 9731915 A | 1-8 |
| Y | JP 2002-255814 A (Sankyo Co., Ltd.),<br>11 September, 2002 (11.09.02),<br>Par. No. [0015]; pharmaceutical preparation example 1<br>& WO 2002/51412 A1 & EP 1350511 A1<br>& US 2004/0024013 A1 & AU 2002217464 A<br>& CN 1491109 A | 1-8 |
| Y | JP 2004-51639 A (Sankyo Co., Ltd.),<br>19 February, 2004 (19.02.04),<br>Par. No. [0024]; pharmaceutical preparation example 1<br>& WO 2004/009119 A1 & EP 1555032 A1<br>& US 2005/0192245 A1 & AU 2003248077 A1<br>& CN 1681533 A | 1-8 |
| Y | JP 2001-346600 A (Roquette Freres),<br>18 December, 2001 (18.12.01),<br>& EP 1138661 A1 & US 2003/0026832 A1<br>& AU 3138201 A & CN 1358703 A<br>& KR 2001-0093759 A | 1-8 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- JP HEI641139 B
- JP 2002145883 A
- JP HEI10310586 B
- JP 2002255814 A
- JP 2003246735 A
- JP 2004051639 A
- WO 2004098713 A

### Non-patent literature cited in the description

- **D. Chulia et al.** Powder Technology and Pharmaceutical Process. Elservier Science Pub Co, 01 December 1993
- **Leon Lachman et al.** The Theory and Practice of Industrial Pharmacy. LEA & FEBIGER, 1986
- **Herbert A. Lieberman et al.** Pharmaceutical Dosage Forms: Tablets. MARCEL DEKKER INC, 1989, vol. 1